# EUROPEAN PATENT APPLICATION

(11) **EP 2 641 975 A1**
(43) Date of publication of application: **25.09.2013**
(21) Application number: 12382104.3
(22) Date of filing: 21.03.2012
(51) Int. Cl.: C12Q 1/68

(54) **Device for detection of analytes in affinity bioassays**

(71) Applicant: Vircell, S.L., 18320 Santa Fe (Granada) (ES)
(72) Inventor: Mendoza Montero, Joaquín, 18008 Granada (ES); Rojas González, Almudena, 18008 Granada (ES); Mendoza López, Pablo, 18008 Granada (ES)
(74) Representative: ZBM Patents

(57) **Abstract**

A device for detection of analytes in affinity bioassays comprising at least one transparent wall portion, at least one test recess adapted to receive at least one test strip and arranged such that said test strip is visible through the transparent wall portion, at least one cavity adapted to receive a container containing a fluid sample, said cavity being in fluid communication with said at least one test recess, and piercing means arranged in correspondence with said at least one cavity, such that upon insertion of a container in the cavity the container is pierced and at least a portion of the sample is released from the container and reaches the test recess. The invention further relates to the use of such a device for detection of analytes of a nucleic acid amplification reaction and to a detection kit comprising such a device and a test strip.

## Description

The present invention relates to devices for analysing a fluid sample and more particular to a device for detection of analytes in affinity bioassays. The invention further relates to the use of such a device for detection of analytes of a nucleic acid amplification reaction such as Polymerase Chain Reaction (PCR).

Furthermore, the invention relates to a detection kit comprising such a device and a test strip.

### BACKGROUND ART

The analysis of diagnostically relevant biological samples for the detection of infectious pathogens is gaining importance in the recent years. One of the most widely used methods for pathogen identification involves the use of the Polymerase Chain Reaction (PCR) for the amplification of desired nucleic acid fragments that may subsequently be detected using any known assay.

PCR amplification is very well-known for its speed, ease of use, sensitivity and robustness, since poor quality samples may also be analysed. The PCR amplification of specific target nucleic acids, e.g. a gene or a gene fragment of a particular pathogen, is usually followed by detection of the amplified products, for example by means of an affinity/binding bioassay.

By affinity or binding bioassay it is meant a type of assay to detect bioanalytes by specific interaction of a binding moiety with the target bioanalyte, such binding moieties being molecules or molecule segments capable of binding specifically to the target bioanalyte. The nature of the bioanalyte often determines the binding molecule to be used in its detection. Affinity or binding bioassays usually use a solid support wherein the binding moiety is deposited. These binding/affinity assays are well-known assays used in biotechnology.

A well-known drawback of the diagnosis of infectious pathogens by means of PCR is that contamination may easily occur thus leading to false positives. It is known that one of the main causes of false-positive results relates to contamination of the PCR amplification products due to their manipulation prior to or upon detection. It is thus desirable to reduce such a contamination risk.

A degree of reduction of false positives has been obtained by carrying out PCR reactions in closed environments combining the amplification and detection processes. Document US2010291668, for example, discloses such a device. Other systems may comprise an element that allows attachment to the reaction tube, once the PCR is completed, wherein said element and the reaction tube may be connected to each other in a sealed manner.

Furthermore, portable detection devices are also being developed, which maintain the sample in a relatively closed environment, are relatively easy to handle and are relatively inexpensive. EP2048245 and US20090181388, for example, disclose such a device, but this kind of solution can be relatively cumbersome to operate and therefore the detection process may be expensive to automate. Furthermore, such devices are not quite versatile when more than one amplified sample is to be detected, especially at the same time.

Therefore a need exists to provide a device for detection of analytes, and in particular PCR amplification products, in affinity bioassays which is simple to operate and cost-effective while avoiding contamination, and which also allows an easy automation of the detection process.

### SUMMARY

In a first aspect, a device for detection of analytes in affinity bioassays is provided. The device comprises at least one transparent wall portion, at least one test recess adapted to receive at least one test strip and arranged such that said test strip is visible through the transparent wall portion, at least one cavity adapted to receive a container containing a fluid sample, said cavity being in fluid communication with said at least one test recess, and piercing means arranged in correspondence with said at least one cavity, such that upon insertion of a container in the cavity the container is pierced and at least a portion of the sample is released from the container and reaches the test recess.

According to this aspect the device works as a substantially enclosed detection unit in combination with a test strip for detection of the analytes, e.g. analytes of an amplified nucleic acid. Contamination of the sample may be avoided because the container is pierced only once it is inside the device, and the sample is then released on the test strip. Contamination of the sample may further be avoided because inserting the container in the device does not involve opening the device. The device may be made in one piece and when it is made from various pieces it may actually be sealed. The device does not require any special arrangement or manipulation but only the insertion of the container with the sample in the cavity. This way, it is rather simple to operate and the detection process may be easily automated as the only action that needs automation is in one direction, the direction of insertion of the container with the sample into the device. It is thus also a cost-effective solution.

Furthermore, the presence of a transparent wall portion through which the test strip is visible allows reading the results from outside the device.

In some embodiments, the transparent wall portion may be arranged on a side of the device opposite to that in which the at least one cavity may be provided. This way, the device can be easily placed e.g. on a thermic block (thermoblock), whereby the temperature can be relatively easily controlled. This may be of particular interest in implementations in which the detection reaction is a hybridization of DNA wherein the control of temperature may be important.

Moreover, in some implementations in which the test strip may comprise a great number of lines or alternatively a dot-array, the transparent wall portion may be easily placed on a scanner for automatic interpretation of the results.

In some embodiments, the device may comprise two or more cavities adapted to receive a container containing a sample. This way, two or more samples, e.g. samples of PCR amplification products, can be detected at the same time and on the same test strip. In these cases, the detection reaction may be a hybridization of DNA and the test strip may be provided with dried chemical reagents used for the hybridization reaction. These kinds of assays, called "multitest assays", are of special interest in those cases in which it would be desirable to detect more than one PCR amplification products as mixing various PCR amplification products may go, at least in part, against its efficiency. In alternative embodiments, the further cavities may be used for reagents which may help with the detection reaction. This way, upon release of the fluids held in the containers that may be inserted in the cavities, the fluids may contact the test strip simultaneously or in a stepwise manner. These reagents may be used e.g. for improving reaction performance. For example, the use of a stringent wash solution may improve specificity of the test, reducing background signal. In another example, conjugate or developing reagents may be added using the second (or third) cavities after introducing the first container with the sample in the first cavity.

In some of these embodiments comprising two or more cavities, at least two cavities may be arranged at an angle and they may be converged towards the same area of the test recess. This way, the fluids contained in each container inserted in each cavity may react simultaneously on a test strip provided in the test recess. In other embodiments, at least two cavities may be arranged at an angle and they may be directed towards different areas of the test recess. This way, the reactions of the fluids contained in the containers inserted in these cavities may be consecutive. This alternative is especially interesting in those cases wherein the further cavities contain reagents.

In some embodiments, at least one cavity may comprise a bulge portion arranged on a wall of the cavity such that when the container containing a sample is inserted in the cavity, the bulge portion generates a pressure increment inside the container. Such a pressure increment inside the container ensures the release of sample out of the container and towards the test recess once the container is pierced.

In some embodiments, the device may be a single-use disposable device and the discarded unit may be left in a safety place.

Another aspect relates to the use of a device substantially as hereinbefore described for detection of analytes of a nucleic acid amplification reaction.

In a further aspect a detection kit comprising a device substantially as hereinbefore described and a test strip is provided. The test strip may have at least a test area comprising dried chemical reagents used for affinity bioassays.

In some embodiments, the test strip may further comprise a control area and the test area may comprise a pad having the dried chemical reagents used for hybridization reaction. In others, it may also further comprise a control area and the test area may comprise dried chemical reagents used for an immunoassay.

Additional objects, advantages and features of embodiments of the invention will become apparent to those skilled in the art upon examination of the description, or may be learned by practice of the invention.

### BRIEF DESCRIPTION OF THE DRAWINGS

Particular embodiments of the present invention will be described in the following by way of non-limiting examples, with reference to the appended drawings, in which:
Figure 1 shows an exploded view of a device according to a first embodiment;
Figure 2 shows a side view of the device of figure 1;
Figure 3 shows a perspective view of the device of figure 1;
Figure 4 shows a top view of a device according to a second embodiment;
Figure 5 shows an exploded view of the device of figure 4;
Figure 6a and 6b show respectively a front and a side view of the device of figure 4; and
Figure 7 shows bottom view of the device of figure 4.

### DETAILED DESCRIPTION OF EMBODIMENTS

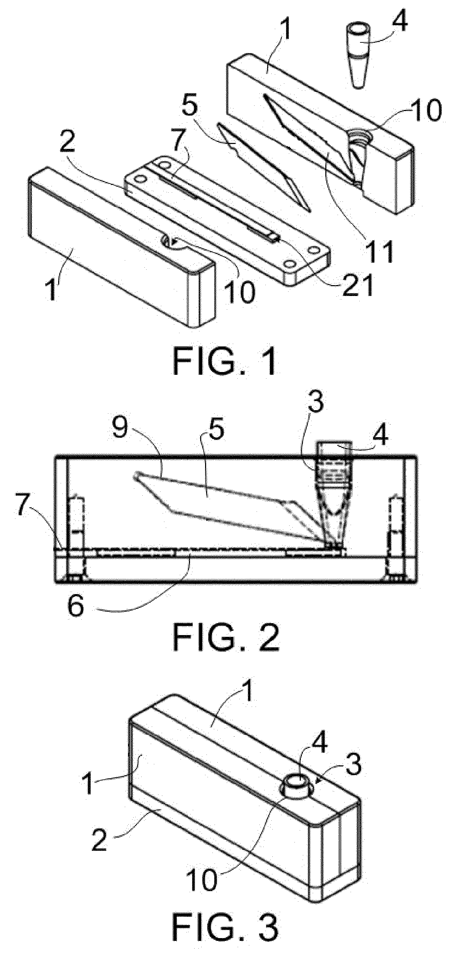

Figure 1 shows an exploded view of a device according to a first embodiment. Figure 1 shows a device that may be made from two first bodies 1 and a cover 2. Each first body 1 may have a first concavity 10 and a second concavity 11. The two first bodies 1 may be joined together with their concavities 10 and concavities 11 matching together respectively (see figure 3). This way a cavity (see figure 3) may be defined by the two first concavities 10 and a housing 9 (see figure 2) may be defined by the two second concavities 11. In such a housing 9 piercing means, e.g. a blade 5, may be arranged.

Moreover the second concavities 11 may be arranged in correspondence with the first concavities 10 such that the blade 5 arranged in the housing 9 may project into the cavity 3 and may pierce a container 4 when it is inserted therein.

Furthermore, figure 1 shows that the cover 2, which may be a transparent cover, may comprise a third concavity (see arrow 21). When the transparent cover 2 is mounted together with the two first bodies 1, such a third concavity 21 may define a test recess (see figure 2) in fluid communication with the cavity 3 defined by the two first concavities 10. A test strip 7 may be arranged in the test recess. The test strip 7 may be arranged such that it may be visible from outside the device through the transparent cover 2. In alternative implementations more than one test strip may be arranged in the test recess. It should be understood that in other implementations other ways of forming the test recess may be foreseen, e.g. protrusions from the cover.

Figure 2 shows a side view of figure 1 wherein a first body has been made transparent in order to show the interior of the device. Figure 2 shows that the test recess 6 may be in fluid communication with the cavity 3 and that the blade 5 may pierce a bottom portion of a container 4 inserted in the cavity 3. This way, at least a portion of sample is released from the container 4 and reaches a test strip 7 that may be arranged in the test recess 6.

Figure 3 shows a perspective view of figure 1. Figure 3 shows a cavity 3 that may be defined by the two first concavities 10. A container 4 containing a fluid sample may be inserted in the cavity 3.

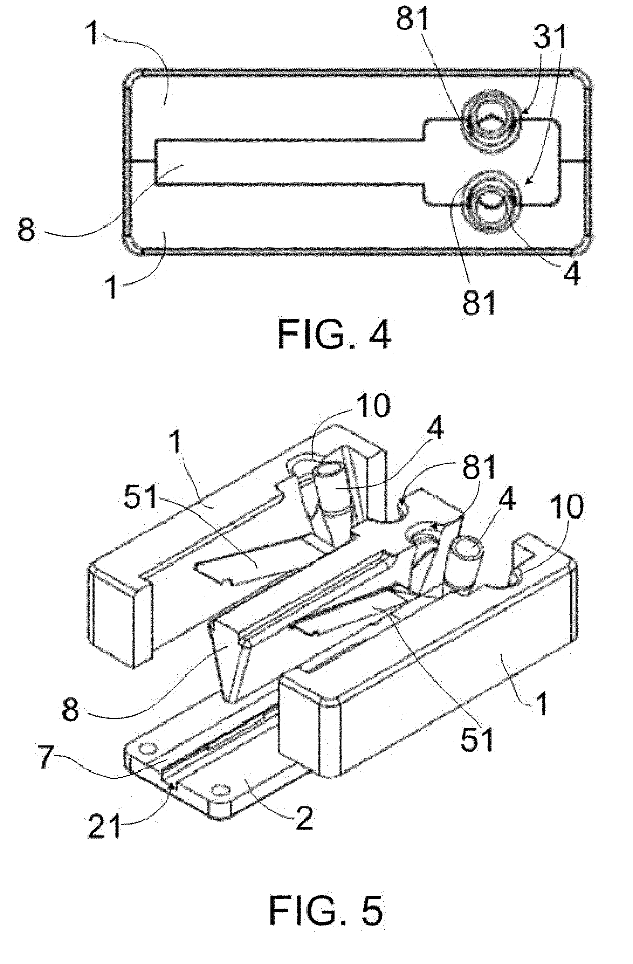

Figure 4 shows a top view of a device according to a second embodiment. The same reference numbers will be used for matching parts. Figure 4 shows a device that may have two cavities 31 adapted to receive a container 4 containing a fluid sample. Differently from the example of figure 1, the example of figure 4 shows a device that may comprise a second body 8 that may be mounted in between the two first bodies 1. Such a second body 8 may have two fourth concavities 81. Each fourth concavity 81 may match with a first concavity of the first bodies 1. This way, when the bodies 1 and 8 are mounted together with the cover (see figure 5), two cavities 31 may be defined instead of only one as in the example of figure 1. It should be understood that in other implementations more concavities may be present defining more cavities. The device may also be made in one piece or from various pieces/bodies substantially as hereinbefore explained.

Figure 5 shows an exploded view of figure 4. Figure 5 shows a second body 8 that may comprise two fourth concavities 81. Each fourth concavity 81 together with each first concavity 10 provided in the two first bodies 1 can define a cavity (See figure 4). Figure 5 shows that the fourth concavities 81 may each be in correspondence with a blade 51 such that when a container 4 is inserted in each cavity 31 a blade 51 can project into each cavity 31 and pierce a bottom portion of each container 4.

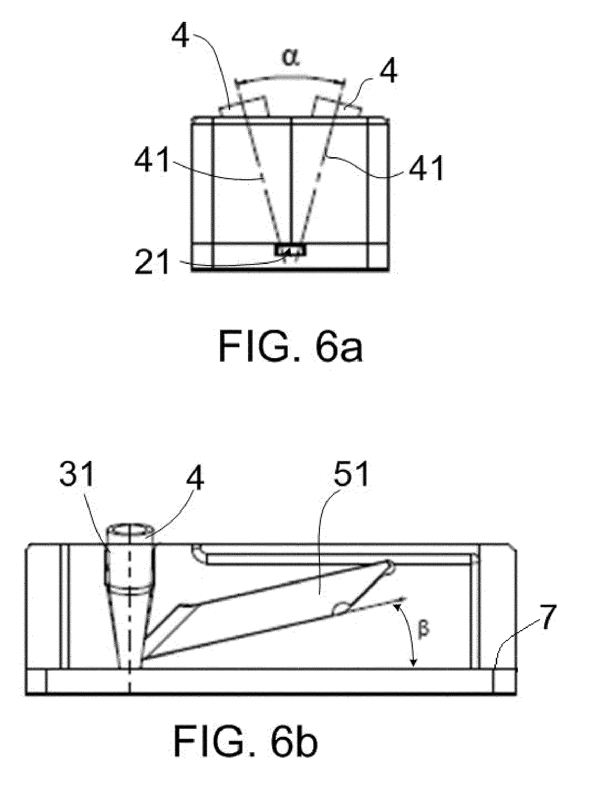

Figure 6a and 6b show a front and a side view of figure 4 respectively wherein the first bodies have been made transparent in order to show the interior of the device.

Figure 6a shows two containers 4 that may be inserted in the two cavities (see figure 4). A symmetry axis 41 of each container 4 shows that when they are inserted in the cavities they may be arranged at an angle α and may be converged towards the same portion of the third concavity 21 provided in the cover 2 wherein a test strip may be arranged. This way, two different samples contained in two different containers 4 inserted in the two cavities may flow towards the same area and may react simultaneously on the test strip 7. In a preferred embodiment, in an example of a device having two cavities, the angle α may be around 30°.

In alternative embodiments, the two cavities may be arranged at an angle and may be directed towards different areas of the test recess wherein a test strip may be arranged. This way, the reactions of the fluids contained in the containers inserted in the cavities may be consecutive and a sample may be reacted with different reagents consecutively.

Figure 6b shows that the blades 51 (only one is shown) are arranged with an inclination β from the horizontal. The blades 51 may thus pierce a bottom portion of a container 4 inserted in each cavity 31. This way, at least a portion of sample is released from the container 4 and reaches a test strip 7 that may be arranged in the test recess. In a preferred embodiment, in an example of a device having two cavities, the angle β may be around 13°.

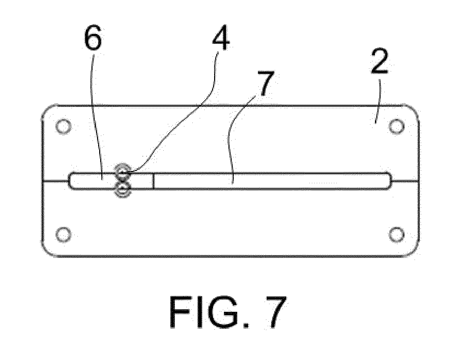

Figure 7 shows a bottom view of the device of figure 4. The test strip 7 may be visible from outside the device through the transparent cover 2. Furthermore, figure 7 shows the two containers converging towards the same portion of the test recess 6. The cover 2 may be a flat surface with no protuberances therein and it may thus be easily placed on a scanner (not shown) for automatic interpretation of the results. In alternative embodiments, other types of readers may be used, portable or not, or the scanner may be combined with a computer which may provide quantitative or qualitative results.

Furthermore, the cover may be easily placed e.g. on a thermic block (thermoblock), whereby the temperature can be relatively easily controlled. This may be of particular interest in implementations in which the detection reaction is a hybridization of DNA wherein the control of temperature may be important.

In some of above embodiments, the bioanalyte to be detected may be a DNA or a RNA molecule. In some of these cases it may be the amplification product of a PCR reaction. In this case, the binding molecule may often be a hybridization probe, i.e. a fragment of DNA or RNA of variable length (usually 100-1000 bases long), which may detect the presence of a target sequence that is complementary to the sequence in the probe. The probe thereby hybridizes to nucleic acid (DNA or RNA) whose base sequence allows probe-target base pairing due to complementarity between the probe and the target.

In others, the bioanalyte to be detected may be a protein and the binding molecule to be used can thus be an antibody. Affinity bioassays that use antibodies or molecules that have antibody-like functions, such as aptamers, are generally called immunoassays. A typical example of an immunoassay is an Enzyme-Linked ImmunoSorbent Assay (ELISA).

In some embodiments, a device substantially as hereinbefore described may further comprise a bulge portion arranged on a wall of the at least one cavity. This way, when the container containing sample is inserted in the cavity the bulge portion may generate a pressure increment inside the container. The size of the at least one cavity may depend on the size of the container containing the sample that will be used. This way, some pressure may have to be exerted on the container in order to fit it in the at least one cavity.

In some embodiments, the container containing a sample may be any closed container such as micro centrifuge tubes e.g. a container with a conical bottom and an integral snap cap usually known as Eppendorf. An advantage of a device substantially as hereinbefore described used in combination with a container such as an Eppendorf relates to avoidance of the need to open the container with the sample after e.g. the amplification reaction is carried out and prior to or upon detection. The device only needs the Eppendorf containing the amplified sample to be inserted in the at least one cavity. This minimizes the chances of contamination of the sample and, therefore, false positives in the analysis.

In some embodiments, the test strip may be replaced by any support having binding elements such as a substrate having a surface containing one or more synthetic capture units capable of specifically binding with a synthetic binding unit of a target analyte. For example, the substrate may include a plurality of synthetic capture units that are positioned on the substrate so as to form one or more addressable lines. Such a substrate may be placed on the test recess of the device so as to avoid movement of the substrate, though it does not necessarily need to be encased within the test recess. A variety of materials may be used as the substrate; such materials are known to those skilled in the art.

In some embodiments, the device may further comprise a sealing material arranged on part of its outer surface or in between the different bodies forming the device. This may help to ensure its closure.

Although only a number of particular embodiments and examples of the invention have been disclosed herein, it will be understood by those skilled in the art that other alternative embodiments and/or uses of the invention and obvious modifications and equivalents thereof are possible. Furthermore, the present invention covers all possible combinations of the particular embodiments described. Thus, the scope of the present invention should not be limited by particular embodiments, but should be determined only by a fair reading of the claims that follow.

## Claims

1. A device for detection of analytes in affinity bioassays comprising:
- at least one transparent wall portion,
- at least one test recess adapted to receive at least one test strip and arranged such that said test strip is visible through the transparent wall portion,
- at least one cavity adapted to receive a container containing a fluid sample, said cavity being in fluid communication with said at least one test recess, and
- piercing means arranged in correspondence with said at least one cavity, such that upon insertion of a container in the cavity the container is pierced and at least a portion of the sample is released from the container and reaches the test recess.

2. Device according to claim 1, wherein the transparent wall portion is arranged on a side of the device which is opposite to that in which the at least one cavity is provided.

3. Device according to any of claims 1 or 2, comprising two or more cavities adapted to receive a container containing a sample.

4. Device according to claim 3, wherein at least two cavities are arranged at an angle and are converged towards the same area of the test recess.

5. Device according to claim 3, wherein at least two cavities are arranged at an angle and are directed towards different areas of the test recess.

6. Device according to any of claims 1-5, wherein at least one cavity comprises a bulge portion arranged on a wall of the cavity such that when the container containing a sample is inserted in the cavity, the bulge portion generates a pressure increment inside the container.

7. Device according to any of claims 1-6, wherein the piercing means are one or more blades arranged such that they cut a bottom portion of the container.

8. Device according to any of claims 1-7 comprising at least two bodies having concavities defining the at least one cavity adapted to receive a container and the at least one recess for housing the piercing means.

9. Device according to claim 8, further comprising a transparent cover having a further concavity arranged such that the test recess is defined between the two bodies and the further concavity of the transparent cover.

10. Devices according to any of claims 1-9, wherein it is a single-use disposable device.

11. Use of a device according to any of claims 1-10 for detection of analytes of a nucleic acid amplification reaction.

12. Detection kit comprising a device according to any of claims 1-11 and a test strip having at least a test area comprising dried chemical reagents used for affinity bioassays.

13. Detection kit according to claim 12, wherein the test strip further comprises a control area and the test area comprises a pad having the dried chemical reagents used for hybridization reaction.

14. Detection kit according to claim 12, wherein the test strip further comprises a control area and the test area comprises dried chemical reagents used for an immunoassay.
